(19) **Europäisches Patentamt**
**European Patent Office**
**Office européen des brevets**

(11) **EP 1 845 837 B1**

(12) **EUROPEAN PATENT SPECIFICATION**

(45) Date of publication and mention
of the grant of the patent:
**29.05.2013 Bulletin 2013/22**

(21) Application number: **06704094.9**

(22) Date of filing: **20.01.2006**

(51) Int Cl.:
*A61B 5/00* (2006.01)    *G01N 21/64* (2006.01)
*G01N 21/47* (2006.01)    *A61B 5/1459* (2006.01)
*A61B 5/1455* (2006.01)

(86) International application number:
**PCT/CA2006/000080**

(87) International publication number:
**WO 2006/076810 (27.07.2006 Gazette 2006/30)**

(54) **METHOD AND APPARATUS FOR MEASURING CANCEROUS CHANGES FROM REFLECTANCE SPECTRAL MEASUREMENTS OBTAINED DURING ENDOSCOPIC IMAGING**

VERFAHEN UND VORRICHTUNG ZUM MESSEN VON KREBSARTIGEN VERÄNDERUNGEN AUS WÄHREND DES ENDOSKOPISCHEN ABBILDUNGSVERFAHRENS ERHALTENEN REFLEXIONSSPEKTRUMMESSUNGEN

PROCEDE ET APPAREIL POUR MESURER UNE EVOLUTION CANCEREUSE A PARTIR DE MESURES DE REFLECTANCE SPECTRALE OBTENUES PAR IMAGERIE ENDOSCOPIQUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.01.2005 US 646005 P**

(43) Date of publication of application:
**24.10.2007 Bulletin 2007/43**

(73) Proprietors:
• **Verisante Technology, Inc.**
**Vancouver BC V6M 2A3 (CA)**
• **BC Cancer Agency**
**Vancouver, British Columbia V5Z 4E6 (CA)**

(72) Inventors:
• **Zeng, Haishan**
**Vancouver, BC V5W 1 G6 (CA)**
• **Fawzy, Yasser Sherif**
**Vancouver, BC V6J 1H1 (CA)**

(74) Representative: **TBK**
**Bavariaring 4-6**
**80336 München (DE)**

(56) References cited:
WO-A1-2004/110265    WO-A2-2004/106896
US-A- 4 661 706    US-A1- 2004 044 287
US-B2- 6 898 458

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to the field of optical spectroscopy and more particularly to the method for obtaining information about tissue physiology and morphology using diffuse reflectance spectroscopy. The purpose of the invention is to develop a non-invasive optical method for cancer detection.

Lung cancer is the leading cause of cancer death in North America, and it has the second most common cancer incidence among both men and women. Medical research indicates that cancer can be treated more effectively when is detected early, when lesions are smaller or when tissue is in a precancerous stage. Unfortunately, conventional lung endoscopy (bronchoscopy) based on white light reflectance (WLR) imaging, which is used typically to detect the cancer lesions in the central airways of the lung, can only detect about 25 percent of the lung cancers. Most of these lesions are in the late stage when cancer has progressed and is fatal. This detection rate has created the need for a detection or imaging modality to accompany WLR imaging and achieve better diagnostic performance for cancer detection.

A number of research groups have investigated the use of tissue autofluorescence to improve the detection sensitivity of cancerous lesions. Just as certain morphological changes in tissue may be associated with disease, chemical changes may also be exploited for disease detection especially for early detection of disease. When tissue is illuminated (or excited) with specific wavelengths of ultraviolet (UV) or visible light, biological molecules (fluorophores) will absorb the energy and emit it as fluorescent light at longer wavelengths (green/red wavelength region). These wavelengths of light are selected based on their ability to stimulate certain chemicals in tissue that are associated with disease or disease processes. Images or spectra from these emissions (fluorescence) may be captured for observation and/or analysis. Diseased tissue has considerably different fluorescence signals than healthy tissue so the spectra of fluorescence emissions can be used as a diagnostic tool.

In United States Published Patent Application No. 2004/245350 to Zeng, entitled *"Methods and Apparatus for Fluorescence Imaging using Multiple Excitation-Emission Pairs and Simultaneous Multi-Channel Image Detection",* the inventor reports use of a second independent fluorescence signal in the red/NIR wavelength region. The diseased tissue such as cancerous or pre-cancerous tissue illuminated with the red/NIR light, unlike the tissue properties discussed above, emits fluorescence, providing intensities that are higher for diseased tissue than for normal tissue. These properties may be exploited to improve image normalization and diagnostic utility of images.

Although fluorescence imaging provides increased sensitivity to diseases such as cancer, there are also some trade offs. A commercial fluorescence imaging system has achieved sensitivity of 67 percent for lung cancer detection. However, such increase in detection sensitivity was at the cost of the decreased detection specificity, which was reduced to 66 percent compared to 90 percent for WLR imaging alone. The result was increased medical costs related to the enlarged number of biopsies caused by the increased number of false positives.

In order to provide more accurate diagnosis of cancerous tissue, a more convenient approach has been to perform additional non-invasive and real-time cancer diagnosis that would increase detection specificity, reduce medical cost, and help doctors during surgery to define cancerous region of the tissue. There are few known methods of non-invasive cancer diagnosis, such as reflectance spectroscopy and fluorescence spectroscopy, both of which are based on detection of biochemical and morphological variation of the diseased tissue.

Biological tissue is a turbid medium, which absorbs and scatters incident light. When light impinges on tissue, it is typically multiple elastically scattered but at the same time absorption and fluorescence can occur, too. Further scattering and absorption can occur before light exits the tissue surface containing compositional and structural information of the tissue. This information can be used for detection of pre-cancers and early cancers that are accompanied by local metabolic and architectural changes at the cellular and subcellular level, for example, changes in the nuclear-to-cytoplasm ratio of cells and changes in chromatin texture. These changes affect the elastic scattering properties of tissue.

Reflectance spectroscopy is an analysis of a light reflected from tissue. Tissue reflectance spectroscopy can be use to derive information about tissue chromophores (molecules that absorbs light strongly), *e.g.* hemoglobin. The ratio of oxyhemoglobin and deoxy-hemoglobin can be inferred and used to determine tissue oxygenation status, which is very useful for cancer detection and prognosis analysis. It can also be used to derive information about scatterers in the tissue, such as the size distribution of cell nucleus and average cell density. In many cases quantification of chromophore concentration is desired, and this requires the ability to separate the effects of absorption from those of scattering.

Fluorescence spectroscopy is the analysis of fluorescence emission from tissue. Native tissue fluorophores (molecules that emit fluorescence when excited by appropriate wavelengths of light) include tyrosine, tryptophan, collagen, elastin, flavins, porphyrins and nicotinamide adenine dinucleotide (NAD). Tissue fluorescence is very sensitive to chemical composition and chemical environment changes associated with disease transformation. Exogenous or exogenously-induced chromophores that have been shown to accumulate preferentially in the diseased areas can also be used.

Another type of spectroscopic technique that has been used to examine tissues involved the use of Raman spectroscopy. Raman spectra convey specific information about the vibrational, stretching, and breaking bond energies of the illuminated sample. Raman spectroscopy probes molecular vibrations and gives very specific, fingerprint-like spectral features and has high accuracy for differentiation of malignant tissues from benign tissues. Raman spectroscopy can also be used to identify the structural and compositional differences on proteins and genetic materials between malignant tissues, their pre-cursers, and normal tissues. The development of an *in vivo* tissue Raman probe, however, is technically challenging due to the weak Raman signal of tissue, interference from tissue fluorescence, and spectral contamination caused by the background Raman and fluorescence signals generated in the fiber itself

Another non-invasive imaging technology is optical coherence tomography (OCT). It is based on the principle of low-coherence interferometry where distance information concerning various tissue microstructures is extracted from time delays of reflected signals. OCT can perform high-resolution "optical biopsies" of tissue microstructure *in situ* and in real-time. However, the spatial resolution of commercial OCT systems still cannot meet the clinical requirements for accurate in vivo endoscopy diagnosis.

Other than these methods, there are on-going research studies in the area of non-invasive cancer diagnosis based on morphological alteration of cell structure for cancer cells. One of the most prominent features used by pathologist to diagnose tissue as being cancerous is the presence of enlarged and crowded nuclei. Since the nuclei of cancerous cells are significantly larger than nuclei of normal cells for many cancer types, the target of these research studies is to estimate the average size of scatterers such as nuclei, mitochondria, and other organelles of cells, non-invasively through an optical system.

When a beam of light reaches the tissue under investigation, part of it will be specularly reflected by the surface, while the rest is refracted and transmitted into the tissue. The light transmitted into the tissue will be scattered and absorbed. After multiple scattering, some of the transmitted light will return to the tissue surface and appear for detection. Light scattering by biological tissues are caused by refractive index variations inside the tissue at the boundaries of various microstructures such as cell nucleus and collagen bundles. Thus, tissue scattering property changes with variations in a tissue's microstructure properties and morphology, which are often accompanied with tissue pathological changes. For example, when normal tissue becomes cancerous, the nucleus size of the cells and the epithelial layer thickness increase as does the total volume occupied by the cells (micro-scatterers). Such changes in the tissue microstructure and morphology have been found to cause intrinsic differences in the light-scattering properties of the normal and cancerous lesions.

In particular, two measurement approaches could be identified in literature for obtaining quantitative differences on scattering properties of normal and cancerous lesions using reflectance spectral measurements. One approach is to measure the single light-scattering spectra (LSS) originated from the superficial tissue layers, and to extract quantitative information about the scattering structures at the cellular and sub-cellular levels. The LSS technique examines variations in the elastic scattering properties of cell organelles to infer their sizes and other dimensional information. In order to measure cellular features in tissues and other cellular structures, it is necessary to distinguish the weak, singly scattered light from diffuse light, which has been multiple scattered and no longer carries easily accessible information about the scattering objects. Consequently, concentration of the scatterers in suspension must be low so that information obtained from only angular distribution of single scattered photons can be analyzed. Analysis of the singly-backscattered light spectrum using light-scattering theory provides information about the size and number density of cell nuclei without tissue removal.

Nevertheless, these LSS measurements are limited since LSS does not allow obtaining quantitative information about the absorption properties of the tissue such as chromophore concentration. In addition, LSS measurements are difficult if not impossible to perform during endoscopy applications.

The other approach is to obtain quantitative information about tissue morphology (tissue scattering properties) from the diffuse reflectance spectra (DRS). Diffuse reflectance relies upon the projection of a light beam into the sample where the light is reflected, scattered, and transmitted through the sample material. The back-reflected, diffusely scattered light (some of which is absorbed by the sample) is then collected by the accessory and directed to the detector optics. Only the part of the beam that is scattered within a sample and returned to the surface is considered to be diffuse reflection.

Diffuse reflectance measurements are simpler to implement and allow obtaining quantitative information about the absorption properties as well as the scattering properties. However, in most studies quantitative information obtained from DRS measurements was limited to the estimation of the average bulk tissue optical properties (reduced scattering and absorption coefficient) rather than obtaining quantitative information related directly to tissue microstructure and morphology. This limitation is mainly due to the complex nature of light propagation (multiple scattering) in tissue with such microstructure and morphology. Therefore, it is difficult to characterize the scattering properties at cellular levels from DRS.

Also, the back-reflected light can be considered as derived from two categories, diffuse reflectance and specular reflectance. The specular reflectance is the light that does not propagate into the sample, but rather reflects from the front surface of the tissue. This component contains information about the tissue at the surface. The diffuse component is generally considered more useful for tissue qualification and quantification than is the specular component.

Various approaches, such as using a contact fiber-optic probe, collecting returning radiation over a small collection angle, or using a specular control device, have been proposed to emphasize the diffuse component relative to the specular component. For some tissues, for example, skin, it is relatively easy to obtain such spectra by simply touching the lesion with an appropriate optical fiber bundle that is coupled to a spectrometer. However, for internal organs such as the lung, such set-up would not be practical because of the interferences of the instrument-channel-based fiber probe with biopsy or other therapeutic tools.

Few studies have investigated the potential of diffuse reflectance spectroscopy for detecting tissue cancerous changes. Intrinsic differences on optical properties between malignant and benign lesions/normal tissues were found and were related directly to the changes in tissue physiology and morphology that occurred during cancer transformation. Clinical spectroscopic measurements and analyses have been performed on various organ sites including the lung. In particular, Bard *et al.* have performed spectral measurements and analysis on the abnormal lesions that were identified during fluorescence bronchoscopy and they found significant changes on both absorption-related and scattering-related physiological and morphological properties when tissue became malignant. They have also evaluated the potential of such spectral measurements for improving lung cancer detection specificity. However, their measurements were still conducted using a fiber optic probe inserted through the endoscope instrument channel and been in contact with the tissue surface during the measurement

In principle, DRS as used in the clinical setting is performed in the following manner. An optical fiber probe, fiber-optic bundle, inserted through the biopsy channel of the endoscope and, coupled to a spectrometer, is brought into contact with the tissue surface. The optical fiber probe consists of an illuminating fiber/fiber optical bundle, typically the central core and surrounding fibers/ fiber optical bundles for capturing the returning radiation. Light leaves the illuminating fiber and enters the tissue under investigation. After the processes of scattering and absorption, light that leaves the tissue is captured by the detecting fibers and directed into a spectrometer. The spectrum may than further analyzed to determine the characteristics of the tissue.

Despite the fact that employment of the contact probe geometry gives more controlled diffuse reflectance measurements with less measurement artefacts, the limitation of this kind of measurement is that it is awkward and time consuming for in vivo endoscopic imaging of internal organs.

There is therefore a need for an apparatus to obtain quantitative information about tissue physiological and morphological characteristics directly from diffuse reflectance measurements with the goal for endoscopy applications. Accordingly, the present invention uses a non-contact probe, which eliminates the need for a fiber probe through the instrument channel. Thus, the present invention overcomes the problems presented in the prior art and provides additional advantages over the prior art.

Further prior art referring to this technical field can be found in document WO 2004/110 265 A1, disclosing a "Device for measuring physical properties of the lympanic membrane".

The present invention in one embodiment is a method of obtaining information about tissue from diffuse reflectance spectra according to the method of claim 1.

In another embodiment, the present invention is an apparatus for obtaining information about tissue from diffuse reflectance spectra, according to the corresponding independent claim.

The organization and manner of the structure and operation of the invention, together with further objects and advantages thereof, may best be understood by reference to the following description, taken in connection with the accompanying drawings, wherein like reference numerals identify like elements in which:

FIG. 1 is an illustration of an endoscopy system of the preferred embodiment of the present invention;
FIG. 1A is a cross-sectional view of a probe used in the endoscopy system of FIG.1;
FIG. 1B is a diagram of a spectral attachment used in the endoscopy system of FIG. 1;
FIG. 2 is a diagram of the measuring geometry;
FIG 2a is a graph showing a light fluence distribution $\phi$ as a function of tissue depth z;
FIG. 3 is a diagram of normalization procedure;
FIG. 4 is a diagram of forward model of an example useful for understanding the invention;
FIG. 5 is a flow diagram of the procedure of an embodiment of the invention for disease detection based on the information obtained from the analysis of the diffuse reflectance spectra;
FIG. 6 contains graphs of (a) reflectance spectra measured and fitted (lines) from two normal tissue sites/benign lesions and two malignant lung lesions from the same patient; and (b) corrected true diffuse reflectance spectra derived from Eq. (3) using the correction parameters $a_0$ and $b_0$ obtained with the developed model;
FIG. 7 is a set of graphs showing absorption and scattering related parameters obtained from fitting the two benign and two malignant spectra: (a) Blood volume fraction ($\rho$), (b) Tissue oxygen saturation parameter ($\alpha$), (c) Scattering volume fraction in both layers ($\delta 1$ and $\delta 2$), and (d) Size-distribution parameter describing the scatterer's size-distribution in both layers ($\beta 1$ and $\beta 2$);
FIG. 8 contains graphs of the average reflectance spectra fitted and corrected obtained from the 50 normal tissue/benign lesions versus that of the 50 malignant lesions;

FIG. 9 contains scatter plots of the physiological and morphological parameters of the bronchial mucosa obtained from the reflectance spectral analysis: (a) Blood volume fraction p and (b) Oxygen saturation parameter $\alpha$, (c) Scattering volume fraction $\delta_1$, and (d) Size-distribution parameter $\beta 1$; and

FIG. 10 is a binary plot of (a) blood volume fraction versus the scattering volume, and (b) blood volume fraction versus oxygen saturation parameter.

## DETAILED DESCRIPTION OF THE INVENTION

While the invention may be susceptible to embodiments in different forms, there is shown in the drawings, and herein will be described in detail, specific embodiments and examples useful for understanding the invention with the understanding that the present disclosure is to be considered an exemplification of the principles of the invention, and is not intended to limit the invention to that as illustrated and described herein.

The approach of the present invention is to obtain quantitative information about the absorption-related and/or scattering-related properties from the diffuse reflectance spectra (DRS) obtained during *in vivo* endoscopic imaging. Diffuse reflectance relies upon the projection of a broadband light beam into the sample where the light is absorbed, reflected, scattered, and transmitted or back-reflected through the sample material. The back reflected (back-scattered) light is then collected by the accessory (*e.g.* an optical fiber) and directed to the detector optics. Only the part of the beam that is scattered within a sample and returned to the surface is considered to be diffuse reflection.

Few studies have investigated the potential of diffuse reflectance spectroscopy of tissue for detecting tissue cancerous changes. However, probes in the art used for diffuse reflectance measurements rely upon contact with the tissues to derive data that identify tissue that is suspected of being physiologically changed as a result of pre-cancerous or cancerous activity. Moreover, there has been no clear modelling approach that relates the changes in optical absorption and scattering coefficients to the physiological and morphological parameters to reflect early cancerous changes in real tissues.

Despite the fact that employment of the contact probe geometry gives the most accurate diffuse reflectance measurements, the limitation of this kind of measurement is that it interferences with biopsy and other therapeutic procedures. Accordingly, the present invention uses a non-contact probe, which eliminates the need for a fiber probe through the instrument channel, making clinical applications of this technology much more convenient It also creates measurement geometry of broadbeam illumination and narrow spot detection, simplifying theoretical modelling of the measured spectra.

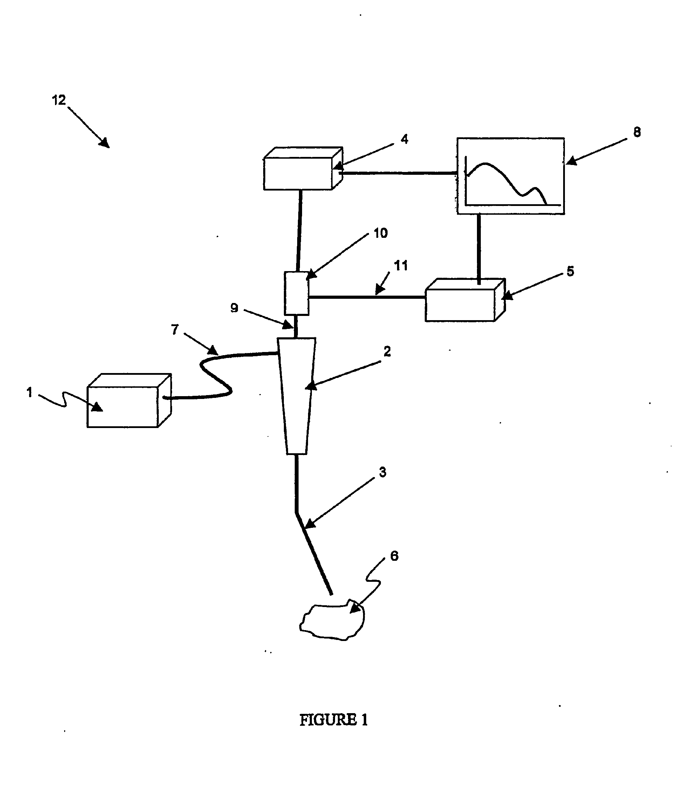

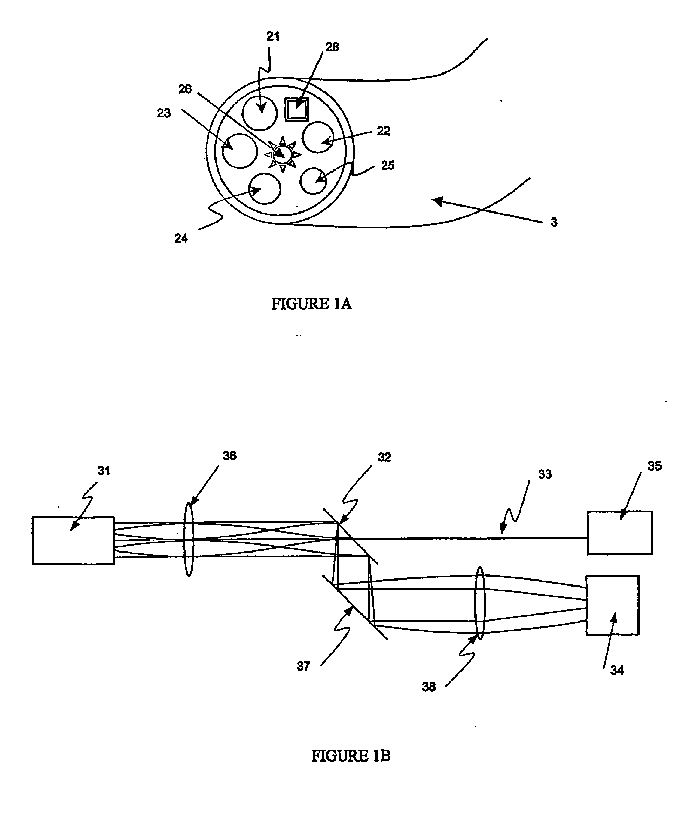

FIG. 1 shows a system 12 for imaging and spectroscopy as is used in the present invention. The system has a light source 1, an endoscope 2 with a probe 3 that is adapted for insertion into the patient, a detection system including an image capture device 4 (such as a camera), a spectral attachment 10, and a spectrometer 5. The light source 1 provides illuminating radiation, preferably broadband light, via an optical fiber bundle 7 to an endoscope 2. The illumination fiber optic bundle 7 extends through the endoscope 2 and probe 3 to direct the illuminating radiation onto an investigated tissue 6.

Light source 1 preferably is a Xenon arc lamp that provides both white light (or light) for white light imaging and reflectance spectral measurements, and a strong blue light (400-450 nm) with a weak near-infrared (NIR) light for fluorescence imaging and fluorescence spectral measurements. The NIR light is employed to form an NIR reflectance image used to normalize the green fluorescence image. (Another embodiment of the present invention employs a second excitation signal for a second fluorescence image used for normalization of the fluorescence image.) The light source 1 could also be a mercury lamp, a tungsten halogen lamp, a metal halide lamp, laser, or LED. Various filters may be added to select a given set of wavelengths.

A processing unit 8 receives data from image-capture device 4 and spectrometer 5, and performs the calculations and processing as described herein. For example, the processing unit 8 will receive the diffuse reflectance spectra, as hereinafter described, and perform the analysis, classifying, and measuring functions described herein. The processing unit 8 is a computer or a microprocessor, preferably a personal computer. The processing unit 8 outputs its results to any output means desired by the user, such as a monitor, an LCD screen, or a printer, or conveys the results to another computer for further analysis, or uses the results for its own internal calculations and analysis.

The returning radiation from the tissue 6, which can be some combination of reflectance light, narrow-band emission light for fluorescence, other narrow bands for normalization, or other types of light, is collected by various lenses and through the imaging bundle 9 is relayed to the detecting system for imaging by image detection device 4 and spectroscopy by spectrometer 5.

Spectral measurements are performed using spectral attachment 10 mounted between the endoscope 2 and the detecting system. An optical fiber 11 carries a fraction of the returning radiation from a spot at an image plane to the spectrometer 5 for spectral analysis.

The distal end of probe 3 is shown in more detail in FIG. 1A. Endoscope probe 3, as shown in cross-section in FIG. 1A, typically contains one or more fiber optic illumination guides 21 to carry the interrogating radiation to the target object (such as the tissue 6 of FIG. 1) and an imaging bundle 22 to carry returning radiation from the tissue 6. Probe 3 also contains an instrument channel 23 for biopsy or other surgical procedures, a water tube 24 for lavage of the target, and an air tube 25 for suction. In addition, the instrument channel 23 may provide access for other medical procedures, such as optical computed tomography, Raman spectroscopy, confocal microscopy, endo-microscopy, laser or drug treatments, gene-therapy, injections, marking, implanting, or other medical techniques. A second imaging modality, such as fluorescence imaging, fluorescence spectroscopy, optical coherence tomography, Raman spectroscopy, confocal microscopy, or white-light reflectance imaging can be combined with the diffuse reflectance spectroscopy modality.

In one embodiment, a light source 26 is placed near the distal end of the endoscope probe 3, as shown in FIG. 1A. For example, by placing at least one LED and preferably at least two LEDs at the end of the endoscope, the fiber optics carrying the illumination or excitation light can be eliminated. LEDs are lower cost, more reliable, longer lasting, lighter in weight, more compact and more efficient than lasers and lamps sources, allowing for better control of imaging and illumination. In addition, a miniature image-capture device 28 can be placed at the distal end of the endoscope 3, as shown in FIG. 1A. This configuration eliminates the need for a fiber optic bundle to channel the returning radiation to the image capture device. Instead, the miniature image-capture device 28 sends signals to a processor such a processing unit 8. This configuration provides an opportunity for increased resolution and improved imaging. In yet another embodiment the illumination sources, image detectors and other expensive optics may be disposed on a removable tip which can be changed from patient to patient, as described in detail in United States Patent Application No. 2006/217594, *"Endoscopy Device with Removable Tip"*

FIG. 1B shows the spectral attachment used in the endoscope system of the present invention. An endoscope 31 employing this spectral attachment 10 is described in detail in United States Patent 6,898,458, to Haishan *et. al., Methods and apparatus for fluorescence and reflectance imaging and spectroscopy and for contemporaneous measurements of electromagnetic radiation with multiple measuring devices,* the disclosure of which is incorporated herein by reference. This patent discloses various devices and configurations for simultaneous white-light and fluorescence imaging along with spectroscopy measurements.

The light coming out from the illuminated tissue 6 of FIG. 1 is focused by lens 36 to form an interim image at the fiber-mirror assembly 32. The centre of the mirror is modified by drilling a hole in the center and an optical fiber 33 is inserted in the hole to take that fraction of the image to a spectrometer 35. The fiber position is seen in the image as a black spot indicating exactly where the spectral analysis will be carried out. The optical fiber 33 carries the reflectance signal from the spot of the image plane (point spectral measurement), which corresponds to an area of 1mm diameter at the tissue surface when the endoscope probe's tip is ten mm away from the tissue surface to the spectrometer 35 for spectral analysis. The physician can align the black spot with the area of interest and the spectral measurement along with the still image is saved into the computer memory. The video image and the spectrum (either in the WLR mode or in the FL mode) are simultaneously displayed on the computer monitor in live mode. Processing of returning radiation through multiple modalities, such as white-light imaging and fluorescence imaging, or imaging and spectrometry, is described in detail in the above-mentioned patent application.

A mirror 37 is placed in parallel to fiber-mirror assembly 32 to turn the light beam back to its original direction and then through lens 38 to a camera 34 for image acquisition. Mirrors 32 and 37 are at an angle of 45 degrees to the incoming light beam for illustration purposes only.

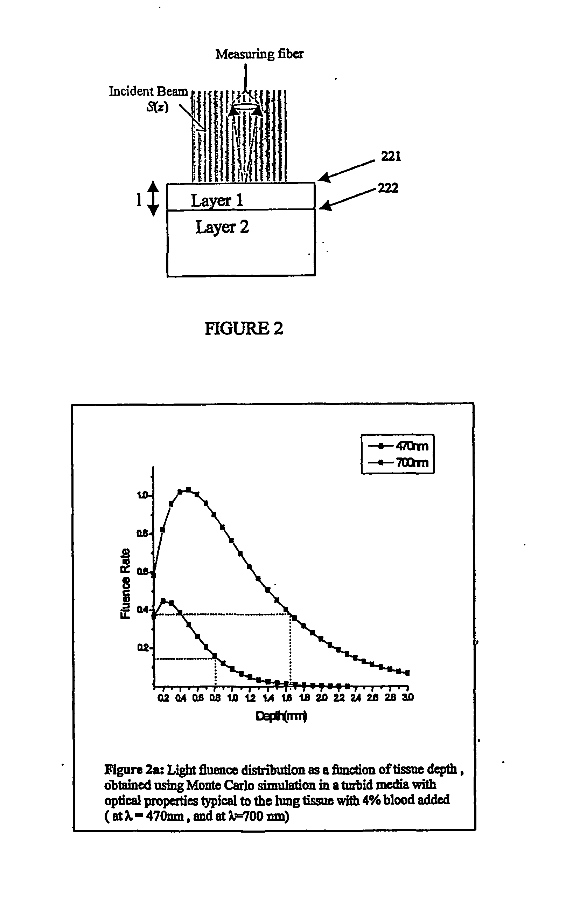

The reflectance measurements performed by the system 12 of the present invention can be represented by an equivalent 1-D measurement geometry shown in FIG. 2. In such geometry, a continuous wave plane source irradiates the tissue and the reflectance is detected from a narrow spot on the tissue surface. The tissue, represented as a two-layer turbid media, is illuminated with a broadbeam S(z) of interrogating radiation through a non-contact perpendicular fiber optical bundle 7. The diameter of the illumination beam is approximately a two-cm spot on the tissue 6. When the tip of the endoscopy probe 3 is ten-mm from the tissue surface 6, for said diameter of the fiber bundle 7, we calculated that the returning radiation or reflectance signal is detected from a one-mm spot at the tissue surface 6.

The method for diffuse reflectance measurement of the present invention will now be described.

The *in vivo* reflectance signal $I_{ml}(\lambda)$ measured from the tissue can be described as following:

$$I_{ml}(\lambda) = a_1 I(\lambda) + b_1 I(\lambda) R_{tm}(\lambda) \qquad (1)$$

where $I(\lambda)$ is the instrument spectral response, including source spectral features, fiber-bundle transmittance, and detector efficiency; $a_1$ is a constant related to the efficiency by which the tissue-surface specular reflection was collected by the probe; $b_1$ is a constant related to the efficiency by which diffuse reflectance from tissue is collected by the measuring probe; and $R_{tm}(\lambda)$ is the true tissue diffuse reflectance to be derived.

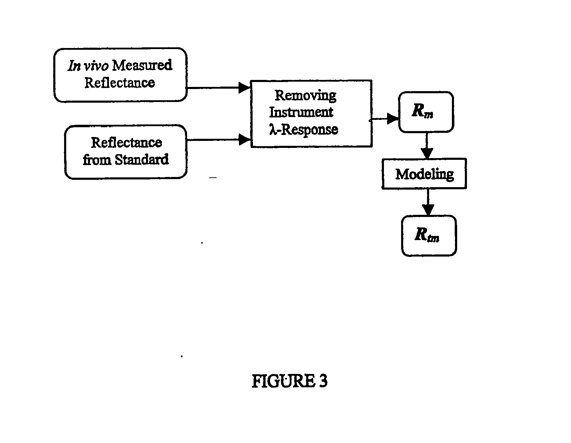

In order to remove an instrument response, normalization should be performed using a standard reflectance disk with known reflectance. The process of removing of the instrument response is also shown diagrammatically in FIG. 3.

The reflectance signal measured from the standard disc $I_{m2}(\lambda)$, can be described as follows:

$$I_{m2}(\lambda) = a_2 I(\lambda) + b_2 I(\lambda) R_s \qquad\qquad (2)$$

where $a_2$ is a constant related to the efficiency by which the specular reflection is collected by the probe; $b_2$ is a constant related to the efficiency by which the diffuse reflectance is collected; and $R_s$ is the reflectivity of the standard disc, which is a constant across the whole visible wavelength range and is very close to one.

The *in vivo* reflectance signal $I_{m1}(\lambda)$ measured from tissue is divided by the reflectance signal $I_{m2}(\lambda)$ measured from the reflectance standard disc to account for instrument spectral response $I(\lambda)$. Dividing Eq. 1 and Eq. 2 and rearranging the equation produces the following:

$$R_m(\lambda) = \frac{I_{m1}(\lambda)}{I_{m2}(\lambda)} = a_0 + b_0 R_{tm}(\lambda) \qquad\qquad (3)$$

where, $R_m(\lambda)$ is the reflectance spectra measured by the apparatus 12 after removing the instrument response, $R_{tm}(\lambda)$ is the true tissue diffuse reflectance spectra, and $a_0$ and $b_0$ are additive offset and multiplicative factors respectively, which depend on the measurement conditions during each *in vivo* measurement performed. This includes the amount of specular reflection collected, the standard disc material used as reference, and the probe distance from the tissue during measurement

We have performed *in vivo* measurements on normal bronchial mucosa and both benign and malignant bronchial mucosa lesions on 22 patients and have obtained a total of 100 spectra. A biopsy sample was obtained for each measurement to classify each measured tissue site into normal, benign or malignant. The pathology examination of biopsies revealed that 21 reflectance spectra were from normal tissue sites, 29 from benign lesions (26 hyperplasia and three mild dysplasia), and 50 from malignant lesions (seven small cell lung cancer, three combined squamous cell carcinoma, 30 non-small cell lung cancer, ten adenocarcinoma). Our analysis was to develop algorithms to differentiate the spectra into two groups

Group One: malignant lesions for tissue pathology conditions that were moderate dysplasia or worse; and

Group Two: normal tissue/benign lesions for tissue pathology conditions that were better than moderate dysplasia.

This binary classification is also in consistent with clinical practice that Group One lesions should be treated or monitored, while Group Two conditions could be left unattended. Also during routine clinical endoscopy examination, any suspected malignant lesions (Group One) should be biopsied, while Group Two conditions will not be biopsied. However, in this specially designed study, for each patient an extra biopsy was taken randomly from either a normal-looking area or a suspected benign lesion so that we can assess the performance of the spectral diagnosis independent of the performance of the imaging diagnosis.

We developed a method and device for tissue classification using quantitative information about tissue physiological and morphological changes obtained from the analysis of diffuse reflectance spectra. In order to achieve this we develop a forward model that relates tissue optical properties (absorption coefficient, scattering coefficient and scattering anisotropy) to the diffuse reflectance (computed) and than an Inversion algorithm to extract the quantitative information about tissue physiological and morphological properties from the tissue diffuse reflectance.

**Forward model (example useful for understanding the invention)**

The forward model is developed in the frame of light transport theory and discrete particle theory that relate computed diffuse reflectance Rc to specific tissue physiological and morphological parameters related to cancer changes. It is known that reflectance depends on the optical properties of the medium and the measurement conditions, such as distance from the probe, and angle in each measurement. For each tissue, light distribution in tissue can be described as a function of an absorption coefficient, a scattering coeffcient, and a scattering anisotropy (direction of scatter). Therefore, we developed an absorption model with an optical absorption coefficient expressed in terms of the micro-vascular absorption-related parameters and a scattering model with a scattering coefficient expressed in terms of the tissue microstructure scatter-related parameters.

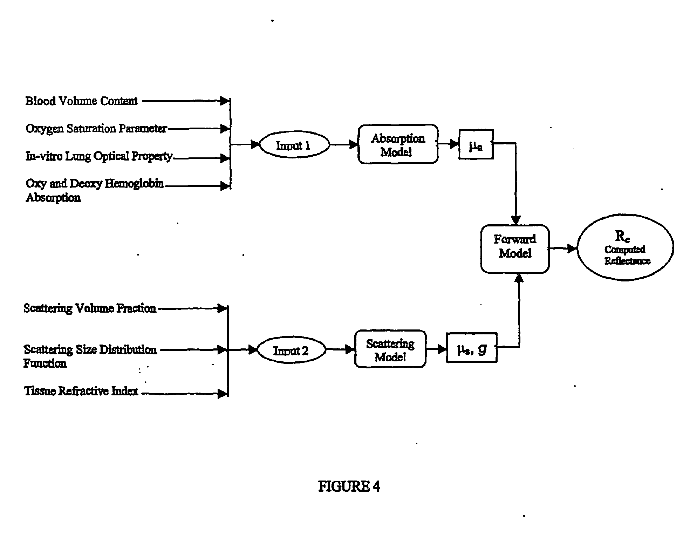

FIG. 4 represents a block diagram of the forward model. The optical absorption coefficient (Input1) is expressed in terms of blood volume content, oxygen saturation, *in vitro* lung optical properties (for example, the absorption coefficient of lung tissue measured *in vitro* with blood drained out) and oxy- and deoxy-hemoglobin absorption. The scattering coefficient (Input2) is expressed in terms of scattering volume fraction, scattering size distribution function and tissue refractive index. All listed parameters are optical properties and are wavelength dependent.

The present example models a system with known optical parameters (absorption and scattering parameters) and calculates a computed value of the diffuse reflectance signal ($R_c$) in relation to those parameters. The light propagated in the tissue is modeled using the general diffusion approximation model used to analyze diffuse back-reflected light with estimated optical coefficients of tissue, which are correlated to morphological structure and chemical composition of the tissue.

Theoretically, the forward model used to describe tissue reflectance spectra $R_c(\lambda)$ at each wavelength, can be obtained using Fick's law:

$$Rc(\lambda) = \frac{-j(z,\lambda)}{I_0}\bigg|_{z=0} = \gamma^{-1}\nabla\phi(z,\lambda)\big|_{z=0} \qquad (4)$$

where $\phi$ is the light fluency spatial distribution, $j$ is the diffuse flux, $I_0$ is the incident power, and $\gamma$ is the diffusion constant, which depends on the tissue optical properties. The light fluency $\phi$ was obtained from the general diffusion approximation modeL The general diffusion equation is different from the standard diffusion approximation model in that it explicitly includes the collimated source in the radiance approximation and it uses the 8-Eddington approximation to model the single scattering phase function, and thus was expected to give better predictions of the visible light (470-700 nm) distribution in the superficial layer of lung, which was found to have low *albedo* value (*i.e.*, $\mu_a \sim \mu_s$).

For a continuous wave plane source decaying exponentially in the z-direction, the general diffusion model is given by:

$$\nabla^2\phi(z) - \kappa_d^2\phi(z) = -\gamma S(z) \qquad (5)$$

$$\kappa_d^2 = 3\mu_a\mu_{tr}; \quad \gamma = -3\mu_s^{\bullet}(\mu_{tr} + g^{\bullet}\mu_t^{\bullet})$$

where $\phi$ is the fluency rate; $S(z)$ is the incident collimated source term, $\mu_{tr}$ is the transport attenuation coefficient equivalent to [$\mu_a + \mu_s$ (1-g)], where $\mu_a$ and $\mu_s$ are absorption and scattering coefficients respectively; $\mu_t^{\bullet}$ is the total attenuation coefficient and is equivalent to $[\mu_a + \mu_s^{\bullet}]$; $\mu_s^{\bullet}$ is the reduced scattering coefficient which is equivalent to $\mu_s(1\text{-}f)$, where $f$ is the fraction of light scattered forward in the $\delta$-Eddington approximation to the scattering phase function; and $g^*$ denotes the degree of asymmetry in the diffuse portion of the scattering. The values of $f$ and $g^*$ were related to the reduced single scattering anisotropy $g$ from the matching of the second moment of the $\delta$-Eddington phase function to the Henyey-Greenstein phase function, and are equivalent to $g^2$ and $\dfrac{g}{1+g}$ respectively.

Since we are interested in the diffuse reflectance that is more affected by the tissue mucosa superficial layer (approximately a 0.5-mm thickness), within which most of the early cancerous changes occur, we solved equation (5) for the two-layer tissue geometry (FIG.2) with the top layer thickness $l$ = 0.5 mm.

The light fluence distribution $\phi$ as a function of tissue depth z (FIG.2a) is obtained using Monte Carlo simulation. The optical properties of lung tissue used in this simulation are obtained from Qu et al. described in detail in Optical Properties of Normal and Carcinomatous Bronchial Tissue, 33 Appl. Opt. 7397-405 (1994) , and a four percent blood volume content is added into the tissue model. The fluence $\phi$ becomes insignificant (reduced by factor e$^{-1}$) for depths after 0.8 and 1.6 mm for $\lambda$ = 470 nm and $\lambda$ = 700nm respectively. Therefore, the measured reflectance signal comes from a tissue volume starting from the surface and up to a depth of 0.8 to 1.6mm depending on the wavelength of the light

We solved Equation (5) in the z-direction for the (1-D) approximation model and the two-layer geometry, for layer 1 and layer 2, using the index mismatching boundary conditions at interface 221 (air-tissue interface) and the index matching boundary conditions at interface 222 (between the two tissue layers), as shown in FIG. 2. By substituting the solution of equation (5) into equation (4) we obtained an expression for the diffuse reflectance spectrum $Rc(\lambda)$ in terms

of the absorption coefficient $\mu_a$, the scattering coefficient $\mu_s$, and the scattering anisotropy $g$.

The absorption coefficient $\mu_a$, is modeled in terms of the blood contents and absorption coefficient of lung tissue measured *in vitro* with blood drained out. Two parameters that are used to describe the blood contents in tissue are the blood volume fraction $\rho$, and the blood oxygen saturation $\alpha$. The absorption properties of lung tissue *in vivo* can be described by the following equations:

$$\mu_a(\lambda) = \mu_{blood}(\lambda)\rho + \mu_{in\ vitro}(1-\rho),$$
$$\mu_{blood}(\lambda) = \alpha\mu_{HbO2} + (1-\alpha)\mu_{Hb} \tag{6}$$

where $\mu_{HbO2}$ and $\mu_{Hb}$ are the absorption coefficients for oxy-hemoglobin and deoxy-hemoglobin respectively. The *in vitro* absorption coefficients $\mu_{in\ vitro,}$ is obtained from *the in vitro* lung tissue measurements made previously by Qu et al. described in detail in Optical Properties of Normal and Carcinomatous Bronchial Tissue, 33 Appl. Opt 7397-405 (1994).

The scattering coefficient $\mu_s$ and the scattering anisotropy g are modeled in terms of the microstructure scatterer volume fractions and size distribution. The tissue scattering model is developed using the fractal approach, assuming that the tissue microstructures' refractive index variations can be approximated by a statistically equivalent volume of discrete micro-scattering particles with a constant refractive index but different sizes.

For micro-scattering particles that are assumed to be spherical in shape, we can calculate the transport scattering coefficients for a bulk tissue by adding randomly the light waves scattered by each particle together. Thus, the transport scattering coefficient $\mu_s$ and the scattering **anisotropy g** can be modeled using the following integral equations:

$$\mu_s(\lambda) = \int_0^\infty [Q(x,n,\lambda)]\frac{\eta(x)}{\upsilon(x)}dx \tag{7}$$

$$g(\lambda) = \frac{\int_0^\infty [g(x,n,\lambda)Q(x,n,\lambda)]\dfrac{\eta(x)}{\upsilon(x)}dx}{\int_0^\infty [Q(x,n,\lambda)]\dfrac{\eta(x)}{\upsilon(x)}dx} \tag{8}$$

where $Q(x)$ is the optical scattering cross section of individual particle with diameter $x$, refractive index $n$ and wavelength $\lambda$; $\upsilon(x)$ is the volume of the scattering particle with diameter $x$ and $g(x)$ is the mean cosine of the scattering angles of single particle with diameter $x$. For spherical micro-particles, $Q(x)$ and $g(x)$ are calculated from Mie-theory using the Mie-scattering code described in details in C.F. Bohren and D.R. Huffman, ABSORPTION AND SCATTERING OF LIGHT BY SMALL PARTICLES, the disclosure of which is incorporated herein by reference.

The volume fraction distribution $\eta(x)$ is assumed to follow a skewed logarithmic distribution:

$$\eta(x) = \delta C_0 x^{-\beta} \exp\left(-\frac{(\ln x - \ln x_m)^2}{2\sigma_m^2}\right) \tag{9}$$

where $\delta$ is the total volume fraction of all the scattering particles in tissue, $\beta$ is the size-distribution parameter (fractal dimension) which determines the shape of the volume-fraction size distribution and is related directly to the size of the scattering particles, $x_m$ and $\sigma_m$ set the center and width of the distribution respectively, and $C_0$ is a normalizing factor obtained from the condition $\delta = \int_0^\infty \eta(x)dx$. The value **of $x_m$** is assumed equal to the geometrical mean of 0 (0.05 μm) and (20 μm) which represent the limits of the scattering particles' range of diameters found typically in tissues. Thus $x_m$ = $[(0.05)(20.0)]^{1/2}$ = 1.0. The width parameter $\sigma_m$ is assumed to be a constant of 2.0 to match with the fractal scaling range of tissues. Having $x_m$ and $\sigma_m$ being set, the larger the value of $\beta$, the higher the contribution of the smaller size particles in the scattering particle size distribution function.

The refractive index of the background surrounding media ($n_{bkg}$) is assumed to be 1.36. The refractive index of the scatterers inside the lung tissue is estimated based on the type of the tissue using the following relation:

$$n = n_{bkg} + f_f(n_f - n_s) + (1 - f_f)(n_n - n_c),$$

$$(10)$$

where $n_f$ is the refractive index of the collagen fibers which is equivalent to 1.47; $n_n$ is the refractive index of the nuclei which is equivalent to 1.4; and $n_s$ and $n_c$ are the refractive index of the interstitial fluids and the intracellular fluids which are equivalent to 1.34 and 1.36 respectively. The fibrous-tissue fraction $f_f$ value is assumed to be ten percent for the first layer (epithelial layer and part of the upper submucosa), and is assumed to be 70 percent for the second layer, which is the lower submucosa and the cartilage layer. These assumptions result in refractive indexes of ($n_1$=1.41) and ($n_2$=1.45) for the first and the second layers respectively.

**Inversion Algorithm** (embodiment of the invention) The Inversion (fitting) algorithm based on a Newton-type iteration scheme is developed to extract information about the tissue absorption and scattering parameters, taking into account measuring conditions (geometry correction parameters $a_0$, $b_0$), from the measured reflectance spectra. The Inversion consists of simulations with the different sets of absorption and scattering parameters to determine the computed diffuse reflectance and determining tissue diffuse reflectance for a different set of geometry correction parameters ($a_0$, $b_0$) and than comparison between the simulations and the tissue diffuse reflectance. When we get the best match it allows us to determine the correct values of the absorption, scattering, and geometry parameters.

The Inversion algorithm can be described through least-squares minimization function:

$$\chi^2 = \sum_{i=1}^{m} \left[ R_m(\lambda_i) - (a_0 + b_0 Rc(\lambda_i)) \right]^2$$

$$(11)$$

where $R_m(\lambda_i)$ is the reflectance measured at wavelength $\lambda_i$; $Rc(\lambda_i)$ is the computed diffuse reflectance at wavelength $\lambda_i$ according to Eq. (4), $b_0$ is an intensity calibration factor to account for the instrument relative intensity measurements, and $a_0$ is an additive factor to account for the specular reflectance collected by the instrument probe during *in vivo* measurements. The following parameters are used as free-fitting variables during the inversion process:

the blood volume fraction ($\rho$) assumed to be the same for both tissue layers,
the blood oxygen saturation parameter ($\alpha$) assumed to be the same for both tissue layers,
the scattering volume fraction in the top layer ($\delta_1$) and in the bottom layer ($\delta_2$),
the size-distribution parameters ($\beta_1$) and ($\beta_2$) for both top and bottom layers respectively, and,
the additive and multiplicative terms (geometry correction parameters) in Eq. 3 ($a_0$) and ($b_0$).

Using the Gradient Base Search (Marquardt-type regularization scheme), we can obtain the updates of these *parameters* from the following system of equations:

$$(\zeta^T \zeta + \nu I)\Delta\tau = \zeta^T [R_m - (a_0 + b_0 R_i)],$$

$$(12)$$

Where $\zeta$ is the Jacobian matrix, $\Delta\tau$ is the vector updates for the eight parameters ($\rho$, $\alpha$, $\delta_1$, $\delta_2$, $\beta_1$, $\beta_2$, $a_0$, $b_0$), I is the identity matrix, and $\nu$ is a scalar or diagonal matrix. The Jacobian matrix $\zeta$ represents the sensitivity of the reflectance coefficients measured on the eight parameters and its elements are computed from the derivatives of Eq. (4) with respect to these eight parameters. The inclusion of $a_0$ and $b_0$ in the fitting are essential to account for the specular reflection component and the back-scattering probe collection efficiency, which vary for each measurement and depend, among others, on the probe-tissue distance and angle in each measurement. Thus, the true tissue diffuse reflectance $\dot{R}_{tm}(\lambda)$ can then be extracted from the measured reflectance spectra $R_m(\lambda)$ measured by the apparatus, using the values of $a_0$ and $b_0$ that are obtained from the fitting procedure and substituting in equation (3). Of course, all other parameters, $p$, $\alpha$, $\delta_1$, $\delta_2$, $\beta_1$ and $\beta_2$ are also derived by this Inversion algorithm.

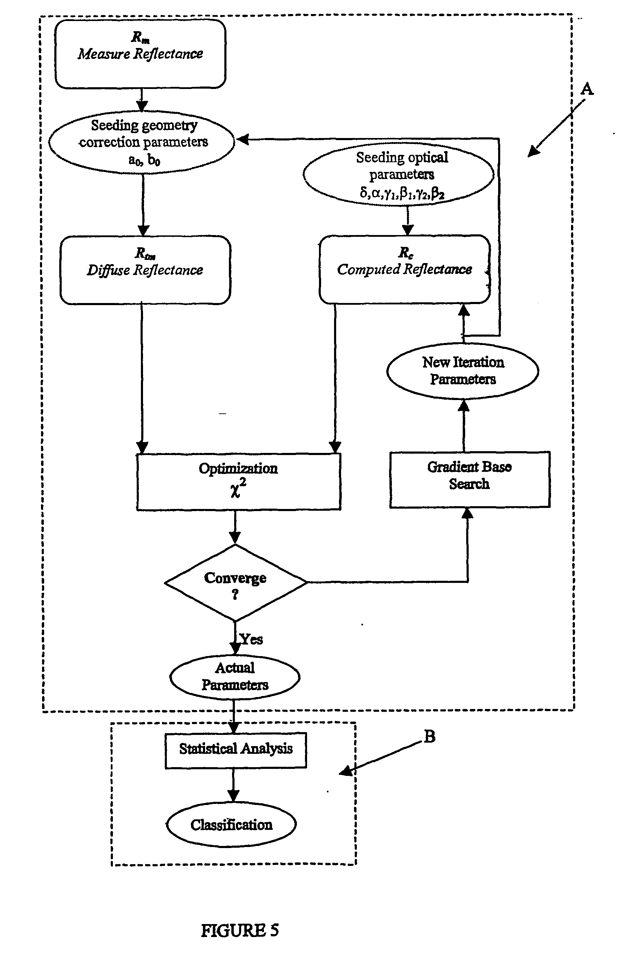

FIG. 5 illustrates the procedure disclosed with the present invention. The forward model and Inversion algorithm described above are used to derive the true diffuse reflectance spectra and to extract the cancer related physiological and morphological properties of the tissue under investigation. We ran a simulation using the inverse algorithm, with a set of known values of tissue optical parameters ($\rho$, $\alpha$, $\delta$, $\beta$) and compared the computed diffuse reflectance with the true tissue diffusion reflectance derived from the measured reflectance for known measuring conditions, (known geometrical correction *parameters* $a_0$, $b_0$). Once we get the best match, we had the actual values of tissue optical *parameters* related to diffuse reflectance. If there is no match, we ran another simulation with new set of parameters. We used a gradient base search to find new iteration parameters.

**Statistical Analysis**

The actual parameters obtained using the Inversion algorithm are used for statistical analysis. The statistical analysis is performed to evaluate the differences in means between the two groups (benign and malignant), to determine which variables discriminate between the two groups, and finally to build classification functions for the evaluation of the developed model predictive classification.

All fitted results obtained from the 100 spectral measurements are collected and saved in groups for statistical analysis. Because we have not been sure if the derived parameters follow normal distributions, the Kolmogorov-Smirnov two-sample test is chosen to evaluate the significance of the differences between the two groups (normal/benign tissue vs. malignant lesions) for each of the six parameters ($\rho$, $\alpha$, $\delta_1$, $\delta_2$, $\beta_1$, $\beta_2$) obtained from our fitting results. Discriminant function analysis (DFA) is then applied to the identified diagnostically significant *parameters* to build diagnostic algorithms for tissue classification. DFA determine the discrimination function line that maximized the variance in the data between groups while minimizing the variance between members of the same group. The performance of the diagnostic algorithms rendered by the DFA models for correctly predicting the tissue status (*i.e.* normal/benign vs. malignant) underlying each parameter set derived from the reflectance spectrum is estimated in an unbiased manner using the leave-one-out, cross-validation method on the whole data set. In this method, one case is removed from the data set and the DFA based algorithm is redeveloped and optimized using data of the remaining cases. The optimized algorithm is then used to classify the withheld spectrum. This process is repeated until all withheld cases (100 spectra/cases) are classified. The sensitivity and specificity are calculated from the results of the classification using the following expressions:

$$\text{Sensitivity} = \% \, (\text{True Positives - False Negatives}) \, / \text{True Positives}$$

$$\text{Specificity} = \% \, (\text{True Negatives - False Positives}) \, / \text{True Negatives}$$

The results of the statistical analysis performed to evaluate the differences in the means of the six measured parameters between benign and malignant group are summarized in Table 1.

**Table 1**

| Parameter | Benign | | Malignant | | |
|---|---|---|---|---|---|
| | Mean | Std. Dev. | Mean | Std. Dev. | Significance (p) |
| $\rho$ | 0.032 | 0.02 | 0.065 | 0.03 | 0.001 |
| $\alpha$ | 0.9 | 0.11 | 0.78 | 0.13 | 0.022 |
| $\delta_1$ | 0.077 | 0.057 | 0.048 | 0.046 | 0.013 |
| $\beta_1$ | 0.97 | 0.15 | 0.91 | 0.12 | 0.095 |
| $\delta_2$ | 0.066 | 0.048 | 0.07 | 0.032 | 0.25 |
| $\beta_2$ | 0.94 | 0.12 | 0.92 | 0.1 | 0.65 |

As shown in the table, the mean value of the blood volume fraction is higher for malignant lesions ($0.065 \pm 0.03$) compared to the benign lesions ($0.032 \pm 0.02$). The mean value of the oxygen saturation parameter is reduced from 0.9 (for benign lesions) to 0.78 for malignant lesions. For the scattering related parameters, the mucosal layer show moderate to significant changes between normal/benign tissues and malignant lesions for the top layer with the mean values of $\delta_1$ and $\beta_1$ for the benign lesions to be 0.077 and 0.97 respectively, compared to 0.048 and 0.91 for the malignant lesions.

The scattering parameters ($\delta_2$ and $\beta_2$) for the bottom layer show minimal differences between the normal/benign tissue and malignant lesions. It should be noted that the larger the value of $\beta$, the higher the contribution of the smaller size particles in the scattering particle size distribution function. Thus, an increase in $\beta$ value indicates a decrease in the scattering particle average size. The statistical analysis, using the Kolmogorov-Smirnov two-sample test, show that the malignant group has significant increase in the blood volume fraction, $\rho$ (p =0.001 <0.05), significant decrease in the oxygen saturation parameter, $\alpha$ (p =0.022 < 0.05), and significant decrease in the mucosa layer scattering volume fraction, $\delta_1$ (p =0.013 < 0.05) compared to the benign group. The results also show moderate significant decrease in the size-distribution parameter of the mucosa layer ($\beta_1$) in the malignant group compared to the benign group (p =0.095 <0.1).

It should be noted that the significant increase in the blood volume fraction of the malignant lesions measured in our study agreed with the biological observations that tumors and cancerous tissues exhibit increased microvasculature and accordingly increased blood content. The significant decrease in the blood oxygenation in the malignant lesions is consistent in that hypoxia-related changes are occurring during cancerous development and could be related to the increase in tissue metabolism rate, the lower quality of the tumoral microcirculation, and to the high proliferation rate of the cancerous cells. The significance decrease in the scattering volume fraction found in the measured malignant lesions is consistent with the results obtained by Bard *et al.* for the lung cancer lesions and the results obtained by Feld *et al.* for the colon polyps. The explanation for such decrease in the scattering volume faction is still poorly understood due to the complex nature of tissue scattering process. However, this decrease may be related to the decrease in the mitochondrial content, which has been found to contribute most significantly to the light scattering in the backward (reflectance) directions or to the changes in the refractive index of the cytoplasm due to an increased protein and enzyme content. The size-distribution parameter ($\beta_1$) decrease means increased scatterer particle sizes on average for malignant tissues as compared to normal/benign tissues. This is consistent with the fact that cancerous cells have larger nuclei than normal and benign cells.

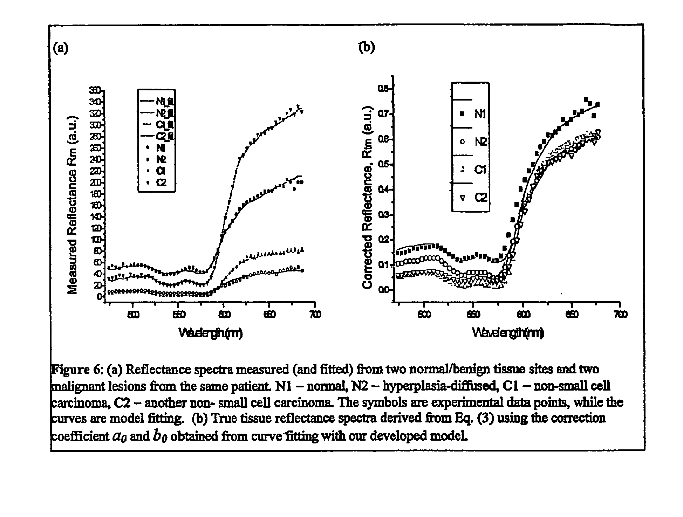

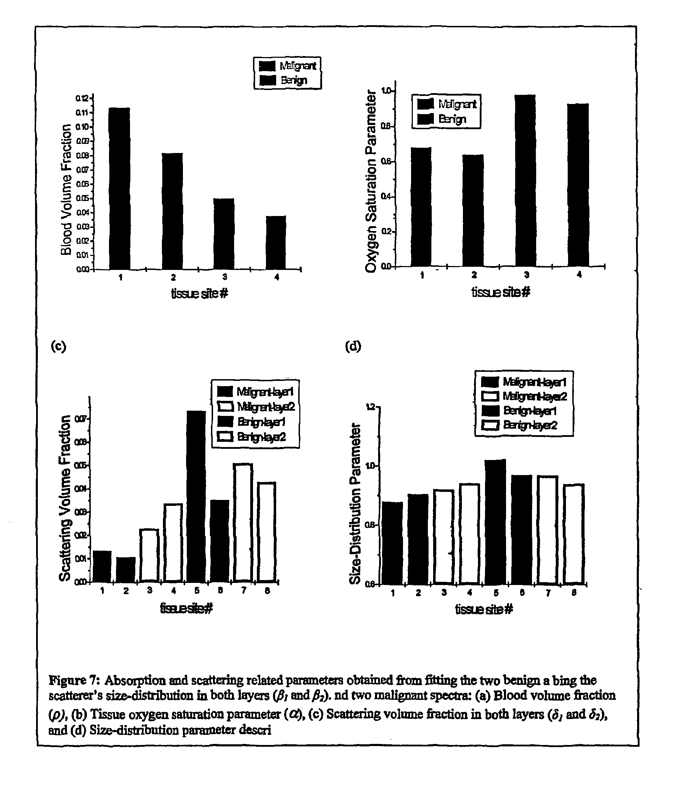

The results obtained by analyzing the 100 reflectance spectra measured in vivo using the above-described model and curve fitting are shown in the FIGS. 6 through 10. An example of the reflectance spectra measured in vivo from two malignant lesions (two non-small cell lesions) and two benign sites (one normal lesion and one hyperplasia-diffused lesion) from the same patient are shown in FIG. 6a. As can be seen, the measured reflectance spectra have large intensity differences, which are related to the variations in the specular reflectance signal and the distance between the endoscope tip and the tissue surface for different measurements. The accuracy of the model fitting to the measured reflectance demonstrated the validity of the developed method. The true tissue diffuse reflectance spectra, $R_{tm}(\lambda)$ is then derived by correcting the in vivo measured reflectance spectra $R_m(\lambda)$ using the fitting results. The corrected true tissue diffuse reflectance spectra, $R_{tm}(\lambda)$, are shown in FIG. 6(b). The specular reflection components have been successfully removed and the reflectance intensities fall between 0 and 1 (or 100 percent), while the original uncorrected spectra, $R_m(\lambda)$, in FIG. 6(a) have quite arbitrary reflectance intensities between 0 and 350 percent The fitting results obtained from the analysis of the two benign and the two malignant spectra are summarized in FIG. 7.

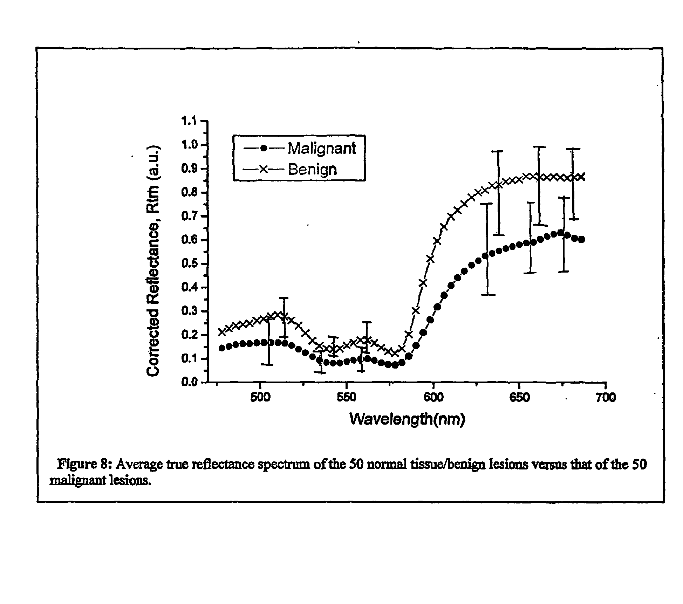

The average of the corrected reflectance spectra ($R_{tm}$) for both the normal/benign group and the malignant group are shown in FIG. 8. It shows that the average reflectance spectra of the normal/benign group have higher intensities in the measured wavelength range (470 - 700 nm) than the malignant group. This intensity differences are significantly larger for wavelengths above 600 nm. In addition the two hemoglobin absorption valleys around 550 nm and 580 nm are larger and more obvious on the normal/benign group spectral curve than on the malignant group spectral curve. The average fitting parameters ($\rho$, $\alpha$, $\delta_1$, $\beta_1$, $\delta_2$, $\beta_2$) and their standard deviations for the two groups are also shown in Table 1.

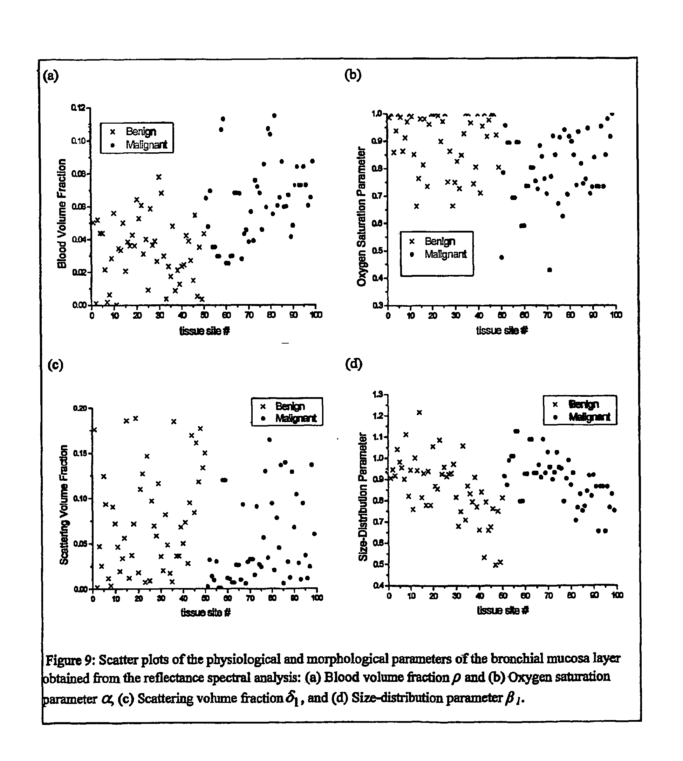

FIG. 9 shows the values of the bronchial mucosa layer (top layer) parameters ($\rho$, $\alpha$, $\delta_1$, $\beta_1$) obtained from the analysis of the 100 benign and malignant reflectance spectra measured.

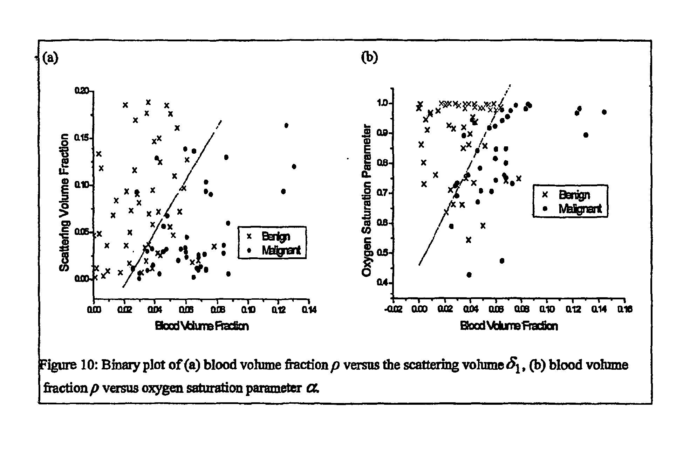

The results obtained from the DFA showed that the three parameters (p, $\alpha$, $\delta_1$) are significant for the discrimination between the two groups. FIG. 10a shows the classification results based on measuring the blood volume fraction ($\rho$) and the scattering volume fraction ($\delta_1$) and (FIG. 10b) shows the classification results based on measuring the blood volume fraction and the tissue oxygen saturation parameters. As shown in the Figures, we can easily identify two domain spaces, with slight overlap, for benign and malignant groups. The DFA results with the leave-one-out, cross-validation method show that we could differentiate the measured lesions into normal/benign and malignant with sensitivity and specificity of 83 percent and 81 percent respectively. Therefore, the method of the present invention, including illuminating tissue 6 with broadband interrogating radiation, collecting the returning radiation from the tissue 6 with a non-contact probe 3, measuring the diffuse reflectance spectra from the returned radiation and analysing measured diffuse reflectance spectra using the steps described above, results in classifying the tissue as benign or malignant with improved sensitivity and specificity.

Please note that the method described above can also be used in conjunction with one or more of the imaging modalities described previously.

While preferred embodiments of present invention are shown and described, it is envisioned that those skilled in the art may devise various modifications of the present invention without departing from the scope of the claims.

**Claims**

1. A method of obtaining information about tissue physiology and morphology from a measured diffuse reflectance spectra, **characterized by**:

   transmitting said measured reflectance spectra to a processing unit (8);
   wherein the processing unit (8) performs the steps of seeding values of geometry correction parameters (a0, b0), seeding values of optical parameters ($\alpha$, $\beta$, $\gamma$, $\delta$),
   calculating a computed diffuse reflectance spectra ($R_c$) with said seeded values of said optical parameters using a one-dimensional light transportation modelling;
   determining a true diffuse reflectance spectra ($R_{tm}$) from a measured reflectance spectra for the seeded values of the geometry correction parameters;
   analyzing the computed diffuse reflectance ($R_c$) and the true diffuse reflectance ($R_{tm}$) using an inverse modeling technique by iteratively fitting the values of the geometry correction parameters and the values of the optical parameters and comparing the correspondingly computed diffuse reflectance ($R_c$) and the true diffuse reflectance ($R_{tm}$) until a match is obtained between the computed diffuse reflectance spectra ($R_c$) and the true diffuse reflectance spectra ($R_{tm}$) to obtain at least one optical property of the tissue from the true diffuse reflectance spectra ($R_{tm}$); and
   deriving information about a physiology and a morphology of the tissue from said at least one optical property.

2. The method of claim 1, wherein said at least one optical property comprises at least one of an optical absorption coefficient, a scattering coefficient, and a scattering anisotropy.

3. The method of claim 2, wherein said absorption coefficient being expressed in terms of blood contents and in vitro tissue optical parameters.

4. The method of claim 3, wherein said absorption coefficient is at least one of an oxygen saturation and a blood volume fraction.

5. The method of any of claims 2 to 4, wherein said scattering coefficient is at least one of a mucosa layer scattering volume fraction and a mucosa layer size-distribution parameter.

6. The method of any of claims 1 to 5, further comprising classifying the tissue as one of benign and malignant based on said optical property.

7. The method of claim 6, wherein said classifying step further comprises comparing said at least one optical property to a data set of known pathology.

8. The method of claim 7, wherein said comparing step comprises using statistical analysis.

9. An apparatus for obtaining information about tissue physiology and morphology from diffuse reflectance spectra, comprising:

   - means for illuminating (1) a tissue with a broadbeam radiation to produce returning radiation;
   - a non-contact probe (3) to measure said returning radiation;
   - means for measuring said reflectance spectra (5) of said returning radiation;
   **characterized by**
   - means (8) for seeding values of geometry correction parameters ($a_0$, $b_0$),
   - means for seeding values of optical parameters ($\alpha$, $\beta$, $\gamma$, $\delta$),
   - means for calculating a computed diffuse reflectance spectra (Rc) for values of said optical parameters;
   - means (8) for determining a true diffuse reflectance spectra ($R_{tm}$) from said measured reflectance spectra for values of said geometry correction parameters;
   - means for analyzing (8) the computed diffuse reflectance ($R_c$) and the true diffuse reflectance ($R_{tm}$) using an inverse modeling technique by iteratively fitting the values of the geometry correction parameters and the values of the optical parameters and comparing the correspondingly computed diffuse reflectance ($R_c$) and the true

diffuse reflectance ($R_{tm}$) until a match is obtained between the computed diffuse reflectance spectra ($R_c$) and the true diffuse reflectance spectra ($R_{tm}$) to obtain at least one optical property of the tissue from the true diffuse reflectance spectra ($R_{tm}$); and
- means (8) for deriving information about a physiology and a morphology of the tissue from said at least one optical property.

10. The apparatus of claim 9, wherein said at least one optical property comprises at least one of an optical absorption coefficient, a scattering coefficient, and a scattering anisotropy.

11. The apparatus of claim 10, wherein said absorption coefficient being expressed in terms of blood contents and in vitro tissue optical properties.

12. The apparatus of claim 11, wherein said absorption coefficient is at least one of an oxygen saturation and a blood volume fraction.

13. The apparatus of any of claims 10 to 12, wherein said scattering coefficient is at least one of a mucosa layer scattering volume fraction and a mucosa layer size-distribution parameter.

14. The apparatus of any of claims 9 to 13, wherein said geometry correction parameters account a specular reflectance collected by said non-contact probe and a variable collection efficiency of said non-contact probe.

15. The apparatus of any of claims 9 to 14, further comprising means for classifying the tissue as one of benign and malignant based on said tissue optical property.

16. The apparatus of claim 15, wherein said classifying means further comprises comparing means for comparing said at least one optical property to a data set of known pathology.

17. The apparatus of claim 16, wherein said comparing means is adapted to use statistical analysis.

18. The apparatus of any of claims 9 to 17, further comprising means (4, 5) for at least one other modality for obtaining information about tissue physiology and morphology.

19. The apparatus of claim 18, wherein said at least one other modality is chosen from the group consisting of fluorescence imaging, fluorescence spectroscopy, optical coherence tomography, Raman spectroscopy, confocal microscopy, or white-light reflectance imaging.

20. The apparatus of any of claims 9 to 19, further comprising means for imaging the tissue with fluorescence imaging comprising at least a first excitation signal for a first fluorescence image and a second excitation signal for a second fluorescence normalization image.

21. The apparatus of any of claims 9 to 20, wherein said means (4, 5) for measuring is adapted to measure said reflectance spectra from substantially a point in an image plane of said returning radiation.

22. The apparatus of any of claims 9 to 21, wherein said illumination means (1) comprises at least one of a Xenon arc lamp, a mercury lamp, a tungsten halogen lamp, a metal halide lamp, a laser, and a light-emitting diode.

23. The apparatus of claim 22, wherein said illumination means (1) further comprises wavelength filters.

24. The apparatus of any of claims 22 and 23, wherein said illumination means (1) is at a distal end of said probe.

25. The apparatus of any of claims 9 to 21, wherein said probe (3) further comprises a removable tip and wherein said illumination means (1) is on said removable tip.

26. The apparatus of any of claims 9 to 25, further comprising at least one light guide coupling said illumination means (1) to said probe (3).

27. The apparatus of any of claims 9 to 26, wherein said probe (3) is an endoscope.

**28.** The apparatus of any of claims 9 to 27, wherein said means for measuring (4, 5) reflectance spectra comprises at least a spectrometer.

**Patentansprüche**

**1.** Verfahren zum Erhalten einer Information über eine Gewebephysiologie und -morphologie aus einem gemessenen diffusen Reflexionsspektrum, **gekennzeichnet durch**:

Übertragen des gemessenen Reflexionsspektrums an eine Verarbeitungseinheit (8);
wobei die Verarbeitungseinheit (8) die Schritte durchführt:

Setzen von Werten von geometrischen Korrekturparametern (a0, b0),
Setzen von Werten von optischen Parametern ($\alpha$, $\beta$, $\gamma$, $\delta$)
Berechnen eines berechneten diffusen Reflexionsspektrums ($R_c$) mit den gesetzten Werten der optischen Parameter unter Verwendung eines eindimensionalen Lichttransportmodells;
Bestimmen eines wahren diffusen Reflexionsspektrums ($R_{tm}$) aus einem gemessenen Reflexionsspektrum für die gesetzten Werte der Geometriekorrekturparameter;
Analysieren der berechneten diffusen Reflexion ($R_c$) und der wahren diffusen Reflexion ($R_{tm}$) unter Verwendung einer inversen Modelliertechnik **durch** iteratives Anpassen der Werte der Geometriekorrekturparameter und der Werte der optischen Parameter und Vergleichen der entsprechend berechneten diffusen Reflexion ($R_c$) und der wahren diffusen Reflexion ($R_{tm}$) bis eine Übereinstimmung zwischen dem berechneten diffusen Reflexionsspektrum ($R_c$) und dem wahren diffusen Reflexionsspektrum ($R_{tm}$) erhalten wird, um zumindest eine optische Eigenschaft des Gewebes aus dem wahren diffusen Reflexionsspektrum ($R_{tm}$) zu erhalten; und
Ableiten einer Information über eine Physiologie und eine Morphologie des Gewebes aus der zumindest einen optischen Eigenschaft.

**2.** Verfahren nach Anspruch 1, wobei die zumindest eine optische Eigenschaft zumindest eine aus einem optischen Absorptionskoeffizienten, einem Streukoeffizienten, und einer Streuanisotropie aufweist.

**3.** Verfahren nach Anspruch 2, wobei der Absorptionskoeffizient in Ausdrücken von Blutinhalten und in-vitro optischen Gewebeparametern ausgedrückt wird.

**4.** Verfahren nach Anspruch 3, wobei der Absorptionskoeffizient zumindest einer aus einer Sauerstoffsättigung und einer Blutvolumenfraktion ist.

**5.** Verfahren nach einem der Ansprüche 2 bis 4, wobei der Streukoeffizient zumindest einer aus einer Mukosaschichtstreuvolumenfraktion und einem Mukosaschichtgrößenverteilungsparameter ist.

**6.** Verfahren nach einem der Ansprüche 1 bis 5, ferner mit Klassifizieren des Gewebes als gutartig oder bösartig basierend auf der optischen Eigenschaft.

**7.** Verfahren nach Anspruch 6, wobei der Klassifizierungsschritt ferner Vergleichen der zumindest einen optischen Eigenschaft mit einem Datensatz einer bekannten Pathologie aufweist.

**8.** Verfahren nach Anspruch 7, wobei der Vergleichsschritt ein Verwenden einer statistischen Analyse aufweist.

**9.** Vorrichtung zum Erhalten einer Information über eine Gewebephysiologie und -morphologie aus einem diffusen Reflexionsspektrum mit:

- einer Einrichtung zum Belichten (1) eines Gewebes mit einer Breitstrahlstrahlung, um zurückkehrende Strahlung zu produzieren;
- einer nicht kontaktierende Sonde (3), um die zurückkehrende Strahlung zu messen;
- einer Einrichtung zum Messen des Reflexionsspektrums (5) der zurückkehrenden Strahlung;
**gekennzeichnet durch**
- eine Einrichtung (8) zum Setzen von Werten von Geometriekorrekturparametern (a0, b0),
- eine Einrichtung zum Setzen von Werten von optischen Parametern ($\alpha$, $\beta$, $\gamma$, $\delta$);

- eine Einrichtung zum Berechnen eines berechneten diffusen Reflexionsspektrums ($R_c$) für Werte der optischen Parameter;
- eine Einrichtung (8) zum Bestimmen eines wahren diffusen Reflexionsspektrums ($R_{tm}$) aus dem gemessenen Reflexionsspektrum für Werte der Geometriekorrekturparameter;
- eine Einrichtung zum Analysieren (8) der berechneten diffusen Reflexion ($R_c$) und der wahren diffusen Reflexion ($R_{tm}$) unter Verwendung einer inversen Modelliertechnik **durch** iteratives Anpassen der Werte der Geometriekorrekturparameter und der Werte der optischen Parameter und Vergleichen der entsprechend berechneten diffusen Reflexion ($R_c$) und der wahren diffusen Reflexion ($R_{tm}$) bis eine Übereinstimmung zwischen dem berechneten diffusen Reflexionsspektrum ($R_c$) und dem wahren diffusen Reflexionsspektrum ($R_{tm}$) erhalten wird, um zumindest eine optische Eigenschaft des Gewebes aus dem wahren diffusen Reflexionsspektrum ($R_{tm}$) zu erhalten; und
- eine Einrichtung (8) zum Ableiten einer Information über eine Physiologie und eine Morphologie des Gewebes aus der zumindest einen optischen Eigenschaft.

10. Vorrichtung nach Anspruch 9, wobei die zumindest eine optische Eigenschaft zumindest eine aus einem optischen Absorptionskoeffizient, einem Streukoeffizient und einer Streuanisotropie aufweist.

11. Vorrichtung nach Anspruch 10, wobei der Absorptionskoeffizient in Ausdrücken von Blutinhalten und in-vitro optischen Gewebeeigenschaften ausgedrückt wird.

12. Vorrichtung nach Anspruch 11, wobei der Absorptionskoeffizient zumindest einer aus einer Sauerstoffsättigung und einer Blutvolumenfraktion ist.

13. Vorrichtung nach einem der Ansprüche 10 bis 12, wobei der Streukoeffizient zumindest einer aus einer Mukosaschichtstreuvolumenfraktion und einem Mukosaschichtgrößenverteilungsparameter ist.

14. Vorrichtung nach einem der Ansprüche 9 bis 13, wobei die Geometriekorrekturparameter eine Spiegelreflexion, die durch die nicht kontaktierende Sonde gesammelt wird, und eine variable Sammlungseffizienz der nicht kontaktierenden Sonde berücksichtigt.

15. Vorrichtung nach einem der Ansprüche 9 bis 14, ferner mit einer Einrichtung zum Klassifizieren des Gewebes als gutartig oder bösartig basierend auf der optischen Gewebeeigenschaft.

16. Vorrichtung nach Anspruch 15, wobei die Klassifizierungseigenschaft ferner eine Vergleicheinrichtung zum Vergleichen der zumindest einen optischen Eigenschaft mit einem Datensatz einer bekannten Pathologie aufweist.

17. Vorrichtung nach Anspruch 16, wobei die Vergleichseinrichtung angepasst ist, eine statistische Analyse zu verwenden.

18. Vorrichtung nach einem der Ansprüche 9 bis 17, ferner mit einer Vergleichseinrichtung (4, 5) für zumindest eine andere Modalität zum Erhalten einer Information über eine Gewebephysiologie und -Morphologie.

19. Vorrichtung nach Anspruch 18, wobei die zumindest eine andere Modalität ausgewählt ist aus der Gruppe, die aus Fluoreszenzabbilden, Fluoreszenzspektroskopie, optischer Kohärenztomographie, Raman-Spektroskopie, konfokaler Mikroskopie oder Weißlichtreflexionsabbilden besteht.

20. Vorrichtung nach einem der Ansprüche 9 bis 19, ferner mit einer Einrichtung zum Abbilden des Gewebes mit Fluoreszenzabbilden, die zumindest ein erstes Anregungssignal für ein erstes Fluoreszenzbild und ein zweites Anregungssignal für ein zweites Fluoreszenznormalisierungsbild aufweist.

21. Vorrichtung nach einem der Ansprüche 9 bis 20, wobei die Einrichtung (4, 5) zum Messen angepasst ist, das Reflexionsspektrum aus im Wesentlichen einen Punkt in einer Bildebene der zurückkehrenden Strahlung zu messen.

22. Vorrichtung nach einem der Ansprüche 9 bis 21, wobei die Belichtungseinrichtung (1) zumindest eine von einer Xenonbogenlampe, einer Quecksilberlampe, einer Wolframhalogenlampe, einer Metallhalidlampe, einem Laser, und einer lichtemittierenden Diode aufweist.

23. Vorrichtung nach Anspruch 22, wobei die Belichtungseinrichtung (1) ferner Wellenlängenfilter aufweist.

**24.** Vorrichtung nach einem der Ansprüche 22 und 23, wobei die Belichtungseinrichtung (1) an einem distalen Ende der Sonde ist.

**25.** Vorrichtung nach einem der Ansprüche 9 bis 21, wobei die Sonde (3) ferner eine entfernbare Spitze aufweist, und wobei die Beleuchtungseinrichtung (1) auf der entfernbaren Spitze ist.

**26.** Vorrichtung nach einem der Ansprüche 9 bis 25, ferner mit zumindest einer Lichtführung, die die Belichtungseinrichtung (1) mit der Sonde (3) koppelt.

**27.** Vorrichtung nach einem der Ansprüche 9 bis 26, wobei die Sonde (3) ein Endoskop ist.

**28.** Vorrichtung nach einem der Ansprüche 9 bis 27, wobei die Einrichtung zum Messen (4, 5) eines Reflexionsspektrums zumindest ein Spektrometer aufweist.

**Revendications**

**1.** Procédé d'obtention d'informations sur la physiologie et la morphologie des tissus à partir d'un spectre de réflectance diffuse mesuré, **caractérisé par** le fait de :

transmettre ledit spectre de réflectance mesuré à une unité de traitement (8) ;
où l'unité de traitement (8) exécute les étapes qui consistent à
initialiser des valeurs de paramètres (a0, b0) de correction de géométrie,
initialiser des valeurs de paramètres optiques ($\alpha$, $\beta$, $\gamma$, $\delta$),
calculer un spectre de réflectance diffuse calculée ($R_c$) avec lesdites valeurs initialisées desdits paramètres optiques utilisant une modélisation de transport de lumière unidimensionnelle ;
déterminer un spectre de réflectance diffuse réelle ($R_{tm}$) à partir d'un spectre de réflectance mesuré pour les valeurs initialisées des paramètres de correction de géométrie ;
analyser la réflectance diffuse calculée ($R_c$) et la réflectance diffuse réelle ($R_{tm}$) utilisant une technique de modélisation inverse en ajustant de manière itérative les valeurs des paramètres de correction de géométrie et les valeurs des paramètres optiques et comparer la réflectance diffuse calculée proportionnellement ($R_c$) et la réflectance diffuse réelle ($R_{tm}$) jusqu'à ce qu'une correspondance soit obtenue entre le spectre de réflectance diffuse calculée ($R_c$) et le spectre de réflectance diffuse réelle ($R_{tm}$) afin d'obtenir au moins une propriété optique des tissus à partir du spectre de réflectance diffuse réelle ($R_{tm}$) ; et
dériver les informations sur une physiologie et une morphologie des tissus de ladite au moins une propriété optique.

**2.** Procédé de la revendication 1, dans lequel ladite au moins une propriété optique comprend au moins l'un(e) d'un coefficient d'absorption optique, d'un coefficient de diffusion, et d'une anisotropie de diffusion.

**3.** Procédé de la revendication 2, dans lequel ledit coefficient d'absorption est exprimé en termes de teneurs en sang et de paramètres optiques de tissus in vitro.

**4.** Procédé de la revendication 3, dans lequel ledit coefficient d'absorption est au moins l'une d'une saturation en oxygène et d'une fraction du volume sanguin.

**5.** Procédé de l'une des revendications 2 à 4, dans lequel ledit coefficient de diffusion est au moins l'un(e) d'une fraction de volume de diffusion de couche de muqueuse et d'un paramètre de distribution de taille de couche de muqueuse.

**6.** Procédé de l'une des revendications 1 à 5, comprenant en outre la classification des tissus en tant que bénins et malins sur la base de ladite propriété optique.

**7.** Procédé de la revendication 6, dans lequel ladite étape de classification comprend en outre la comparaison de ladite au moins une propriété optique d'un ensemble de données de pathologie connue.

**8.** Procédé de la revendication 7, dans lequel ladite étape de comparaison comprend l'utilisation d'une analyse statistique.

**9.** Appareil d'obtention d'informations sur la physiologie et la morphologie des tissus à partir du spectre de réflectance diffuse, comprenant :

un moyen pour éclairer (1) un tissu avec un rayonnement à faisceau large afin de produire un rayonnement de retour ;

une sonde sans contact (3) pour mesurer ledit rayonnement de retour ;

un moyen pour mesurer ledit spectre de réflectance (5) dudit rayonnement de retour ;

**caractérisé par**

un moyen (8) pour initialiser des valeurs de paramètres ($a_0$, $b_0$) de correction de géométrie,

un moyen pour initialiser des valeurs de paramètres optiques ($\alpha$, $\beta$, $\gamma$, $\delta$),

un moyen pour calculer un spectre de réflectance diffuse calculée ($R_c$) pour des valeurs desdits paramètres optiques ;

un moyen (8) pour déterminer un spectre de réflectance diffuse réelle ($R_{tm}$) à partir dudit spectre de réflectance mesuré pour des valeurs desdits paramètres de correction de géométrie ;

un moyen pour analyser (8) la réflectance diffuse calculée ($R_c$) et la réflectance diffuse réelle ($R_{tm}$) utilisant une technique de modélisation inverse en ajustant de manière itérative les valeurs des paramètres de correction de géométrie et les valeurs des paramètres optiques et en comparant la réflectance diffuse calculée proportionnellement ($R_c$) et la réflectance diffuse réelle ($R_{tm}$) jusqu'à ce qu'une correspondance soit obtenue entre le spectre de réflectance diffuse calculée ($R_c$) et le spectre de réflectance diffuse réelle ($R_{tm}$) afin d'obtenir au moins une propriété optique des tissus à partir du spectre de réflectance diffuse réelle ($R_{tm}$) ; et

un moyen (8) de dérivation d'informations sur une physiologie et une morphologie des tissus à partir de ladite au moins une propriété optique.

**10.** Appareil de la revendication 9, dans lequel ladite au moins une propriété optique comprend au moins l'un(e) d'un coefficient d'absorption optique, d'un coefficient de diffusion, et d'une anisotropie de diffusion.

**11.** Appareil de la revendication 10, dans lequel ledit coefficient d'absorption est exprimé en termes de teneurs en sang et de propriétés optiques de tissus in vitro.

**12.** Appareil de la revendication 11, dans lequel ledit coefficient d'absorption est au moins l'une d'une saturation en oxygène et d'une fraction du volume sanguin.

**13.** Appareil de l'une des revendications 10 à 12, dans lequel ledit coefficient de diffusion est au moins l'un (e) d'une fraction de volume de diffusion de couche de muqueuse et d'un paramètre de distribution de taille de couche de muqueuse.

**14.** Appareil de l'une des revendications 9 à 13, dans lequel lesdits paramètres de correction de géométrie comptent une réflectance spéculaire collectée par ladite sonde sans contact et une efficacité de collecte variable de ladite sonde sans contact.

**15.** Appareil de l'une des revendications 9 à 14, comprenant en outre un moyen pour classer les tissus en tant que bénins et malins sur la base de ladite propriété optique des tissus.

**16.** Appareil de la revendication 15, dans lequel ledit moyen de classification comprend en outre un moyen de comparaison pour comparer ladite au moins une propriété optique à un ensemble de données de pathologie connue.

**17.** Appareil de la revendication 16, dans lequel ledit moyen de comparaison est adapté pour utiliser une analyse statistique.

**18.** Appareil de l'une des revendications 9 à 17, comprenant en outre un moyen de comparaison (4, 5) pour au moins une autre modalité d'obtention d'informations sur la physiologie et la morphologie des tissus.

**19.** Appareil de la revendication 18, dans lequel ladite au moins une autre modalité est choisie dans le groupe constitué de l'imagerie par fluorescence, spectroscopie par fluorescence, tomographie par cohérence optique, spectroscopie Raman, microscopie confocale, ou imagerie par réflectance en lumière blanche.

**20.** Appareil de l'une des revendications 9 à 19, comprenant en outre un moyen pour imager les tissus avec l'imagerie par fluorescence comprenant au moins un premier signal d'excitation pour une première image par fluorescence et un deuxième signal d'excitation pour une deuxième image de normalisation par fluorescence.

21. Appareil de l'une des revendications 9 à 20, dans lequel ledit moyen de mesure (4, 5) est adapté pour mesurer ledit spectre de réflectance essentiellement à partir d'un point dans un plan image dudit rayonnement de retour.

22. Appareil de l'une des revendications 9 à 21, dans lequel ledit moyen d'éclairage (1) comprend au moins l'une d'une lampe à arc au xénon, d'une lampe au mercure, d'une lampe halogène au tungstène, d'une lampe aux halogénures métalliques, d'un laser, et d'une diode électroluminescente.

23. Appareil de la revendication 22, dans lequel ledit moyen d'éclairage (1) comprend en outre des filtres de longueur d'onde.

24. Appareil de l'une des revendications 22 et 23, dans lequel ledit moyen d'éclairage (1) est à une extrémité distale de ladite sonde.

25. Appareil de l'une des revendications 9 à 21, dans lequel ladite sonde (3) comprend en outre une pointe amovible et dans lequel ledit moyen d'éclairage (1) est sur ladite pointe amovible.

26. Appareil de l'une des revendications 9 à 25, comprenant en outre au moins un guide de lumière couplant ledit moyen d'éclairage (1) à ladite sonde (3).

27. Appareil de l'une des revendications 9 à 26, dans lequel ladite sonde (3) est un endoscope.

28. Appareil de l'une des revendications 9 à 27, dans lequel ledit moyen (4, 5) pour mesurer un spectre de réflectance comprend au moins un spectromètre.

FIGURE 1

FIGURE 1A

FIGURE 1B

Measuring fiber

Incident Beam
S(z)

221

222

1 Layer 1

Layer 2

FIGURE 2

Figure 2a: Light fluence distribution as a function of tissue depth , obtained using Monte Carlo simulation in a turbid media with optical properties typical to the lung tissue with 4% blood added ( at λ = 470nm , and at λ=700 nm)

FIGURE 3

Blood Volume Content ⟶

Oxygen Saturation Parameter ⟶

In-vitro Lung Optical Property ⟶

Oxy and Deoxy Hemoglobin ⟶
Absorption

( Input 1 )  →  [ Absorption Model ]  →  [ $\mu_a$ ]

[ Forward Model ]  →  ( $R_c$ Computed Reflectance )

Scattering Volume Fraction ⟶

Scattering Size Distribution ⟶
Function

Tissue Refractive Index ⟶

( Input 2 )  →  [ Scattering Model ]  →  [ $\mu_s$, $g$ ]

FIGURE 4

FIGURE 5

Figure 6: (a) Reflectance spectra measured (and fitted) from two normal/benign tissue sites and two malignant lesions from the same patient. N1 – normal, N2 – hyperplasia-diffused, C1 – non-small cell carcinoma, C2 – another non- small cell carcinoma. The symbols are experimental data points, while the curves are model fitting. (b) True tissue reflectance spectra derived from Eq. (3) using the correction coefficient $a_0$ and $b_0$ obtained from curve fitting with our developed model.

**Figure 7:** Absorption and scattering related parameters obtained from fitting the two benign a bing the scatterer's size-distribution in both layers ($\beta_1$ and $\beta_2$). nd two malignant spectra: (a) Blood volume fraction ($\rho$), (b) Tissue oxygen saturation parameter ($\alpha$), (c) Scattering volume fraction in both layers ($\delta_1$ and $\delta_2$), and (d) Size-distribution parameter descri

**Figure 8:** Average true reflectance spectrum of the 50 normal tissue/benign lesions versus that of the 50 malignant lesions.

Figure 9: Scatter plots of the physiological and morphological parameters of the bronchial mucosa layer obtained from the reflectance spectral analysis: (a) Blood volume fraction $p$ and (b) Oxygen saturation parameter $\alpha$, (c) Scattering volume fraction $\delta_1$, and (d) Size-distribution parameter $\beta_1$.

EP 1 845 837 B1

Figure 10: Binary plot of (a) blood volume fraction $p$ versus the scattering volume $\delta_1$, (b) blood volume fraction $p$ versus oxygen saturation parameter $\alpha$.

30

**EP 1 845 837 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004245350 A, Zeng
- WO 2004110265 A1
- US 2006217594 A
- US 6898458 B

**Non-patent literature cited in the description**

- **QU et al.** Optical Properties of Normal and Carcinomatous Bronchial Tissue. *Appl. Opt.,* 1994, vol. 33, 7397-405
- **QU et al.** Optical Properties of Normal and Carcinomatous Bronchial Tissue. *Appl. Opt,* 1994, vol. 33, 7397-405